# EUROPEAN PATENT APPLICATION

(11) **EP 4 293 678 A1**
(43) Date of publication of application: **20.12.2023**
(21) Application number: 22179583.4
(22) Date of filing: 17.06.2022
(51) Int. Cl.: G16H 40/20, G16H 50/70

(54) **PREDICTING SURGERY DURATION**

(71) Applicant: Koninklijke Philips N.V., 5656 AG Eindhoven (NL)
(72) Inventor: NIKOLOVA-SIMONS, Mariana, Eindhoven (NL); KELDERMAN, Rikkert, Eindhoven (NL); DJIKIC, Marko, 5656AG Eindhoven (NL); MONTENIJ, Leon, 5656AG Eindhoven (NL)
(74) Representative: Philips Intellectual Property & Standards

(57) **Abstract**

A system and method are provided for generating a predictive model for predicting a surgery duration. The system and method use a feature selection technique to identify a set of features in training data, which set of features is predictive of the surgery duration, train a number of predictive models using the set of features as input and the surgery duration as prediction target, wherein the predictive models include at least a linear predictive model and a non-linear predictive model, and generate an ensemble model which combines at least two of the predictive models. Such an ensemble model may optimally combine linear and non-linear predictions and therefore allow linear and non-linear relationships between features and the surgery duration to be taken into account. Advantageously, more accurate surgery planning may safeguard the health of patients, for example by ensuring that there are sufficient resources available for acute surgeries, or by avoiding that elective surgeries have to be postponed due to a presumed lack of resources.

## Description

### FIELD OF THE INVENTION

The invention relates to a system and computer-implemented method for generating a predictive model for predicting a surgery duration. The invention further relates to a system and computer-implemented method for using the generated predictive model for predicting a surgery duration. The invention further relates to a computer-readable medium comprising instructions to perform any of the computer-implemented methods.

### BACKGROUND OF THE INVENTION

Hospitals and other health care institutions are facing capacity challenges, for example due to increased demand for care and shortage of healthcare workers. It is therefore of importance that resources such as operating rooms (OR), intensive care units (ICU), post anaesthesia care units (PACU) and general ward resources are efficiently used to be able to provide the timely care that patients require. To ensure the efficient use of these resources, it is common to plan the use of the resources in advance to ensure their timely availability for patients.

For example, it is known to plan surgeries in advance to know which resources will be allocated at which time. In such surgery planning, it is common to use a surgeon's average procedure time for their latest n patients as a booking time for future patients that require a similar procedure, with the number n being typically different for different hospitals. While this model may be quite simple and easily understood, the discrepancy (delta) between the planned and the actual surgery duration may be substantial.

Such discrepancies are highly undesirable as inaccurate surgery planning may endanger the health of patients. For example, inaccurate surgery planning may result in insufficient resources being available for patients which require acute surgery, or elective surgeries of patients having to be postponed even though sufficient resources would have been available. It is therefore desirable to obtain a predictive model which more accurately predicts surgery durations.

### SUMMARY OF THE INVENTION

In a first aspect of the invention, a system is provided for generating a predictive model for predicting a surgery duration. The system comprises:
an input interface for accessing medical data, the medical data comprising records of surgeries, wherein a record is indicative of at least a type of surgery and a surgery duration;
a processor subsystem configured to generate a predictive model for predicting the surgery duration by:
   - using a feature selection technique to identify a set of features in training data, which set of features is predictive of the surgery duration, wherein the training data comprises a first part of the medical data;
   - training a number of predictive models using the set of features in the training data as input and the surgery duration as prediction target, wherein the predictive models include at least a linear predictive model and a non-linear predictive model;
   - using a second part of the medical data, evaluating a performance of each the predictive models in predicting the surgery duration, wherein the evaluating of the performance comprises using a performance metric which characterises a time difference between a predicted surgery duration and an actual surgery duration;
   - based on the performance of the predictive models, generating an ensemble model which combines at least two of the predictive models; and
   - outputting the ensemble model for use in predicting the surgery duration.

In a further aspect of the invention, a computer-implemented method is provided for generating a predictive model for predicting a surgery duration. The method comprises:
accessing medical data, the medical data comprising records of surgeries, wherein a record is indicative of at least a type of surgery and a surgery duration;
generating a predictive model for predicting the surgery duration by:
- using a feature selection technique to identify a set of features in training data, which set of features is predictive of the surgery duration, wherein the training data comprises a first part of the medical data;
- training a number of predictive models using the set of features in the training data as input and the surgery duration as prediction target, wherein the predictive models include at least a linear predictive model and a non-linear predictive model;
- using a second part of the medical data, evaluating a performance of each the predictive models in predicting the surgery duration, wherein the evaluating of the performance comprises using a performance metric which characterises a time difference between a predicted surgery duration and an actual surgery duration;
- based on the performance of the predictive models, generating an ensemble model which combines at least two of the predictive models; and
- outputting the ensemble model for use in predicting the surgery duration.

In a further aspect of the invention, a system is provided for using a predictive model to predict a surgery duration. The system comprises:
- an input interface for accessing:
- input data indicative of at least a type of surgery, and
- model data representing a predictive model, wherein the predictive model is an ensemble model as described in this specification;
a processor subsystem configured to use the input data as input to the predictive model to obtain a prediction of the surgery duration as output.

In a further aspect of the invention, a computer-implemented method is provided for using a predictive model to predict a surgery duration. The method comprises:
accessing input data indicative of at least a type of surgery, and model data representing a predictive model, wherein the predictive model is an ensemble model as described in this specification;
using the input data as input to the predictive model to obtain a prediction of the surgery duration as output.

In a further aspect of the invention, a transitory or non-transitory computer-readable medium is provided, the computer-readable medium comprising data representing a computer program comprising instructions for causing a processor system to perform one or more of the methods described in this specification.

The above aspects of the invention involve generating and using a predictive model for predicting a duration of a surgery of a patient. In some aspects of the invention, the predictive model may be generated and used by one and the same system and/or method, while in other aspects of the invention, the predictive model may be generated by a system and/or method which is different from the system and/or method which subsequently uses the predictive model to predict surgery durations.

The predictive model may be generated using medical data which comprises records of surgeries. Such a record may for example be a record in a database pertaining to a particular surgery but may also be a row item in a log of surgeries. Typically, the records may pertain to actual surgeries having been performed in the past, e.g., in a hospital or other health care institution. The records may for example be specific to one particular hospital. For each or at least a subset of the surgeries, the medical data may indicate a type of the surgery and a duration of the surgery. Typically, the duration may be a measured duration of an actual surgery, and may be defined in any suitable manner, for example in minutes, or hours and minutes, or in a number of time blocks of, e.g., 5 or 10 or 15 or 20 minutes each, etc. The type of surgery may for example be indicated by one or more categorizations and/or by one or more labels. Such categorizations and labels may be mutually exclusive or may overlap. For example, one categorization or label may pertain to the medical specialty associated with the surgery, e.g., cardiothoracic surgery. Typically, the categorization or label may also indicate the type of surgery within a particular surgical specialty, e.g., in cardiothoracic surgery, whether the cardiothoracic surgery is a Coronary Artery Bypass Grafting (CABG) or an Aortic Valve Replacement (AVR). Another categorization or label may for example indicate whether the surgery is an elective surgery or an acute surgery.

In accordance with the above measures, a part of the medical data may be used as training data for machine learning. To identify relevant features for the machine learning, a feature selection technique may be applied to the training data to identify a set of features which is predictive of the surgery duration. Such features may at least comprise the type of surgery, and if the medical data contains additional (meta)data characterizing the surgery and/or the patient, one or more features from this additional (meta)data. Having identified a set of features, several predictive models may be trained using the features from the training data as input and the surgery duration as prediction target. Thereby, each predictive model may be trained to predict the surgery duration from the set of features. As also further discussed below, the predictive models may include at least a linear predictive model and a nonlinear predictive model.
Having trained the predictive models, the performance of each predictive model may then be evaluated. For that purpose, another part of the medical data may be used, which part may also be referred to as 'second' or 'test' part' or as 'test data'. The performance itself may be quantified by a performance metric which may be designed to characterize the time difference between the output of the predictive model, being the predicted surgery duration, and the actual surgery duration indicated in the medical data and may be designed to reward smaller or no differences between the actual surgery duration and the predicted surgery duration while penalizing larger differences.

Having quantified the performance of the predictive models, an ensemble model may be generated which may combine at least two of the predictive models. In the selection of the predictive models to be included in the ensemble model, the performance of each individual predictive model may be taken into account. For example, two of the best performing predictive models may be combined into one ensemble model. As is known per se, such an ensemble model may blend and/or aggregate predictions of individual models to obtain an ensemble prediction. The ensemble model may be output, e.g., in the form of model data, to be used to predict the surgery duration. For example, the ensemble model may be used in a surgical planning department to plan allocation of resources for upcoming surgeries in a hospital.

The above measures have the advantage that surgery durations may be more accurately predicted than conventionally, where typically a surgeon's average procedure time for their latest ten patients is used as a booking time for future procedures. Namely, the predictive models are trained on training data which includes at least the type of surgery, which has been found to be a relevant predictor for surgery duration. In particular, it was found that there are linear and nonlinear relations between features in the medical data and the surgery duration. By providing and training several predictive models on the features in the medical data, which models include at least a linear predictive model and a nonlinear predictive model, such linear and nonlinear relations may be modelled by the individual models. By combining individual models into an ensemble model, a single combined output may be obtained, which output may for example optimally combine linear and non-linear predictions. Advantageously, more accurate surgery planning may safeguard the health of patients, for example by ensuring that there are sufficient resources available for acute surgeries, or by avoiding that elective surgeries have to be postponed due to a presumed lack of resources.

Optionally, the performance metric characterises whether the time difference between the predicted surgery duration and the actual surgery duration is positive or negative and a degree of the difference. Conventionally, in data analysis, error measures such as the Root Mean Square Error (RMSE) and Mean Absolute Error (MAE) are used to quantify the performance of regression predictive models. However, these error measures are hard to interpret by non-expert users, such as clinical staff and planners, and may deter such users from actually using the predictive models. Further, these error measures cannot distinguish between positive and negative time difference. By defining and using a performance metric which characterizes the difference between a predicted surgery duration and the actual surgery duration in terms of actual time, e.g., in minutes or in number of time blocks, and by characterizing whether the difference is negative or positive, the performance metric is defined in a way which is not only relevant for the problem at hand but also well-understandable by clinical staff and planners.

Optionally, the performance metric categorizes the time difference into a number of categories, wherein the categories include at least a first category indicating that the predicted surgery duration exceeds the actual surgery duration by a first margin and a second category which indicates that the predicted surgery duration is less than the actual surgery duration by a second margin. By categorizing the performance into several categories, the performance metric takes into account that differences in prediction within a certain range may be considered equally (sub)optimal and may thus be seen as a single category for the purpose of performance evaluation. Advantageously, the performance characterization in several categories may be easier to interpret than the exact differences between actual and predicted surgery duration.

Optionally, in evaluating the performance, a positive time difference between the predicted surgery duration and the actual surgery duration is weighted differently than a negative time difference. From the perspective of patient health, it may be preferred to ensure that sufficient hospital resources are available for acute cases, and therefore, it may be preferred to overestimate rather than to underestimate surgery durations, as in the former case, planners may take action in advance to ensure that sufficient resources are available, while in the latter case, planners may trust that there are sufficient resources available but which may turn out not to be the case. By weighting a negative time difference between the actual surgery duration and the predicted surgery duration, e.g., an overestimation, differently than a positive time difference, this asymmetry may be taken into account. In other words, the performance metric may more heavily penalize an underestimation of the surgery duration by a certain amount than an overestimation of the duration by the same amount.

Optionally, the system further comprises a user interface subsystem comprising a user input interface to a user input device for receiving user input and a display output to a display for displaying output of the system, wherein the processor subsystem is configured to, using the user interface subsystem:
- enable a user to evaluate the performance of a previous predictive model for predicting the surgery duration using the performance metric;
- request a new predictive model to be generated by the system if the performance of the previous predictive model is deemed insufficient by the user or the system.

Optionally, the processor subsystem is configured to, using the user interface subsystem, enable the user to further evaluate the performance of the new predictive model using the performance metric.

Optionally, the processor subsystem is configured to:
- generate two or more ensemble models which each combine at least two of the predictive models;
- using a third part of the medical data, evaluating a performance of the two or more ensemble models in predicting the surgery duration, wherein the evaluating of the performance comprises using the performance metric; and
- based on the performance of the two or more ensemble models, selecting the ensemble model for output.

In some embodiments, two or more ensemble models may be generated, which may each represent a different combination of predictive models. To be able to select one of the ensemble models for output, the performance of the ensemble models may be evaluated on yet another part of the medical data, which part is also referred to as a 'third' or 'validation' part or as 'validation data'. Preferably, the same performance metric is used to evaluate the performance of the ensemble models as was previously used to evaluate the performance of the individual predictive models.

Optionally, a record in the medical data is further indicative of one or more of: an identity of a surgeon, a clinical role of a surgeon, a type of surgical procedure, a surgical urgency, a type of post-surgery bed, an average surgery duration for a type of surgical procedure for a surgeon, and a number of surgical procedures performed during the surgery. Each of these types of data may be used as a feature in the predictive model, e.g., for training and for subsequent use (inference). In this respect, it is noted that the input data during inference may equally comprise one or more of: an identity of a surgeon, a clinical role of a surgeon, a type of surgical procedure, a surgical urgency, a type of post-surgery bed, an average surgery duration for a type of surgical procedure for a surgeon, and a number of surgical procedures performed during the surgery.

Optionally, the medical data further comprises patient data of patients of the respective surgeries. Patient data may also be indicative of surgery duration and may therefore be used as input for training and later use (inference). In this respect, it is noted that the input data during inference may equally comprise patient data.

Optionally, the patient data is indicative of one or more of: an age, a gender, a body-mass index, an American Society of Anaesthesiology (ASA)-score, a number of medications taken, a number of comorbidities, and a creatine level, of a respective patient. Each of the types of data may be used as a feature in the predictive model for training and inference.

Optionally, the processor subsystem is configured to identify the set of features in the training data using multivariate inferential analysis, wherein the processor subsystem is further configured to use univariate inferential analysis as a filter to determine an input set of features to the multivariate inferential analysis, wherein the features of the input set of features are individually predictive of the surgery duration.

Optionally, the predictive models comprise one or more of: a linear regression-based model, a random forest-based model, and a gradient boosting-based model.

Optionally, a record in the medical data is indicative of whether a surgery is an elective surgery or an acute surgery, wherein the processor subsystem is configured to generate different predictive models for predicting the surgical duration of elective surgeries and for predicting the surgical duration of acute surgeries.

It will be appreciated by those skilled in the art that two or more of the embodiments, implementations, and/or optional aspects of the invention described in this specification may be combined in any way deemed useful.

Modifications and variations of the system, the computer-implemented method and/or the computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system for generating and/or using a predictive model for predicting a surgery duration, which system comprises an input interface for accessing medical data, a processor subsystem for generating and/or using the predictive model and a user interface subsystem for displaying a graphical user interface to a user;
Fig. 2 shows functionality of the system partitioned into a surgery duration predictor part which is concerned with generating the predictive model and a surgery duration analyser part which is concerned with analysing performance of predictive models;
Fig. 3A shows, for a particular set of surgeries, the performance of a conventional predictive model in predicting surgery duration of acute surgeries;
Fig. 3B shows, for a particular set of surgeries, the performance of a conventional predictive model in predicting surgery duration of elective surgeries;
Fig. 4A shows, for a particular set of records of surgeries, the number of surgical procedures, with the procedures having been grouped per medical sub-speciality;
Fig. 4B shows the surgery duration for the surgical procedures of Fig. 4A;
Fig. 5 illustrates the feature selection process by illustrating an output of a multivariate analysis performed by the Boruta feature selection algorithm;
Fig. 6A is the same as Fig. 3A, showing the performance of a conventional predictive model in predicting surgery duration of acute surgeries;
Fig. 6B shows the performance of an ensemble model in predicting surgery duration of acute surgeries, the ensemble model combining a linear predictive model and a gradient boosting model by a random forest algorithm;
Fig. 7 shows a computer-implemented method for generating a predictive model of a surgery duration; and
Fig. 8 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 20: data storage
- 22: training data
- 24: test data
- 26: validation data

- 60: display
- 62: display data
- 80: user input device(s)
- 82: user input data

- 100: system for generating and/or using predictive model
- 120: data storage interface
- 140: processor subsystem
- 142-146: data communication

- 160: memory
- 180: user interface subsystem
- 182: display output interface
- 184: user input interface

- 200: surgery duration predictor
- 210: data splitting
- 212: feature selection from data source(s)
- 214: training predictive models
- 216: testing predictive models
- 218: creating ensemble of predictive models

- 220: surgery duration analyser
- 230: quantify deltas between predicted and actual surgery durations
- 232: categorize surgeries based on deltas
- 234: visualize deltas
- 236: select best predictive model

- 300: categories representing deltas between actual surgery duration and predicted surgery duration
- 302: ahead of schedule categories
- 304: on time category
- 306: behind schedule categories
- 310: surgical procedures according to medical subspeciality
- 312: number of surgeries
- 314: surgery duration
- 320: features
- 322: feature importance

- 400: method of generating predictive model
- 410: accessing medical data
- 420: identifying features in medical data
- 430: training predictive models
- 440: evaluating performance of predictive models
- 450: generating and outputting ensemble model
- 500: non-transitory computer-readable medium
- 510: data representing computer program

### DETAILED DESCRIPTION OF EMBODIMENTS

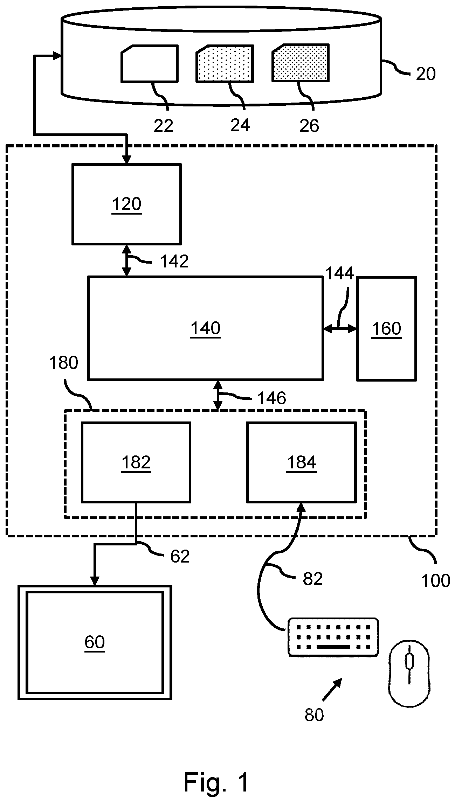

Fig. 1 shows a system 100 which may in some embodiments represent a system for generating a predictive model for predicting a surgery duration, in other embodiments a system for using the predictive model to predict a surgery duration, and in yet other embodiments a system for both generating and using the predictive model.

The system 100 is shown to comprise a data storage interface 120 to a data storage 20. In some embodiments, the data storage 20 may store medical data comprising records of surgeries. As also shown in Fig. 1, the medical data may be split in parts, for example in a first part 22, a second part 24 and a third part 26. As elucidated elsewhere in this specification, the first part 22 may represent training data for training predictive model(s), the second part 24 may represent test data for testing the performance of the predictive model(s), while the third part 26 may represent validation data for use in generating and selecting an ensemble model. Although not shown explicitly in Fig. 1, the data storage 20 may also store other types of data, such as model data representing one or more predictive models, for example in an untrained or trained state, or input data to be used as input to a predictive model during inference. In general, the data storage 20 may serve as short term storage and/or as long-term data storage. In the example of Fig. 1, the data storage interface 120 is shown to be connected to an external data storage 20, such as a network-accessible data storage 20. Alternatively, the data storage 20 may be an internal data storage of the system 100 (not shown in Fig. 1). In general, the data storage interface 120 may take various forms, such as a hard disk or solid-state disk interface to one or more hard disks and/or solid-state disks, or a network interface to a Local Area Network (LAN) or to a Wide Area Network (WAN). In general, the data storage interface 120 may represent an example of an input interface as described elsewhere in this specification.

The system 100 is further shown to comprise a processor subsystem 140 configured to internally communicate with the data storage interface 120 via data communication 142, with a memory 160 via data communication 144 and with a user interface subsystem 180 via data communication 146. The memory 160 may for example be a volatile memory in which a computer program may be loaded which may cause the processor subsystem 140 to carry out functions which are described in this specification, for example in relation to generating a predictive model and/or to using the predictive model.

In some embodiments, the system 100 may comprise a user interface subsystem 180, which user interface subsystem may be configured to, during operation of the system 100, enable a user to interact with the system 100, for example using a graphical user interface. In particular, as also described elsewhere, the graphical user interface may enable the user to analyse the performance of predictive model(s). For that and other purposes, the user interface subsystem 180 is shown to comprise a user input interface 184 configured to receive user input data 82 from one or more user input devices 80 operable by the user. The user input devices 80 may take various forms, including but not limited to a keyboard, mouse, touch screen, microphone, etc. Fig. 1 shows the user input devices to be a keyboard and mouse 80. In general, the user input interface 184 may be of a type which corresponds to the type of user input device(s) 80, i.e., it may be a thereto corresponding type of user device interface. The user interface subsystem 180 is further shown to comprise a display output interface 182 configured to provide display data 62 to a display 60 to visualize output of the system 100. In the example of Fig. 1, the display is an external display 60. Alternatively, the display may be an internal display of the system 100.

In some embodiments, the processor subsystem 140 may be configured to, during operation of the system 100, generate a predictive model for predicting the surgery duration. For that purpose, the processor subsystem 140 may be configured to use a feature selection technique to identify a set of features in the training data 22, which set of features is predictive of the surgery duration, train a number of predictive models using the set of features in the training data as input and the surgery duration as prediction target, wherein the predictive models include at least a linear predictive model and a non-linear predictive model, and use the test data 24 to evaluate a performance of each the predictive models in predicting the surgery duration, wherein the evaluating of the performance comprises using a performance metric which characterises a time difference between a predicted surgery duration and an actual surgery duration. The processor subsystem 140 may be further configured to, based on the performance of the predictive models, generate an ensemble model which combines at least two of the predictive models and output the ensemble model so that it can be used by other systems and methods to predict the surgery duration.

In other embodiments, the processor subsystem 140 may alternatively or additionally be configured to, during operation of the system 100, use the predictive model in form of the ensemble model for inference purposes, namely, to predict the surgery duration from input data. In such embodiments, the processor subsystem 140 may be configured to, using the data storage interface 120, access model data representing the ensemble mode and input data indicative of at least a type of surgery, and to use the input data as input to the predictive model to obtain a prediction of the surgery duration as an output. The predicted surgery duration may for example be output by the system 100 on the display 60.

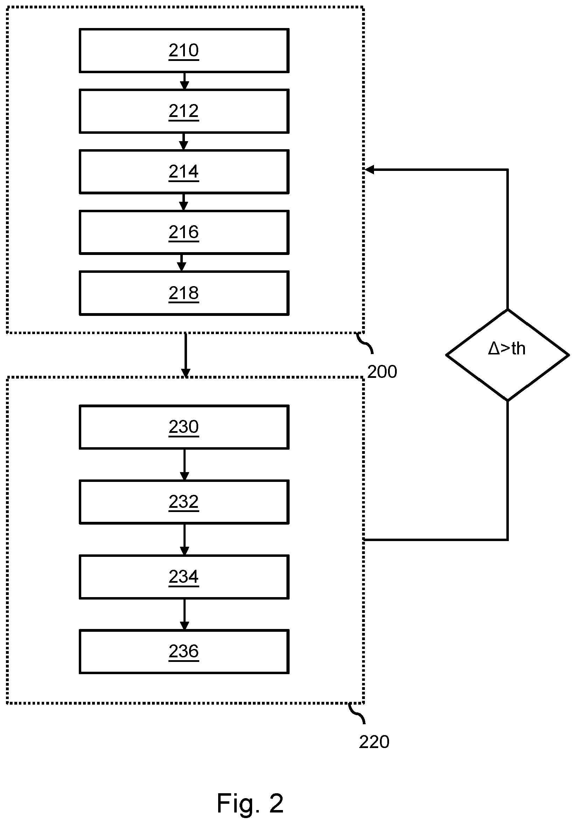

These and other operations of the system 100, and various optional aspects thereof, will be explained in more detail with reference to Fig. 2 et seq.

In general, the system 100 may be embodied as, or in, a single device or apparatus. The device or apparatus may be a general-purpose device or apparatus, such as a workstation or a computer, but may also be application-specific, such as a patient monitor. The device or apparatus may comprise one or more microprocessors which may represent the processor subsystem, and which may execute appropriate software. The software may have been downloaded and/or stored in a corresponding memory, e.g., a volatile memory such as RAM or a non-volatile memory such as Flash. Alternatively, the functional units of the system, e.g., the input interface, the user interface subsystem, and the processor subsystem, may be implemented in the device or apparatus in the form of programmable logic, e.g., as a Field-Programmable Gate Array (FPGA). In general, each functional unit of the system 100 may be implemented in the form of a circuit. It is noted that the system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

Fig. 2 shows functionality of the system 100 of Fig. 1 partitioned into a surgery duration predictor (SDP) part 200 which is concerned with generating the predictive model and a surgery duration analyser (SDA) part 220 which is concerned with analysing performance of predictive models. It is noted that this partitioning is merely optional, in that the functionality of the system may also be differently partitioned or not partitioned at all.

The SDA may be configured to allow the performance of one or more predictive models to be analysed. In an exemplary workflow using the SDA, in a first step 230, an error may be quantified between an actual surgery duration and a surgery duration as predicted by a current predictive model, e.g., as used within the hospital or other health care institution. The current predictive model may for example be a predictive model which was previously generated using machine learning or may be a non-machine learned model which was generated based on heuristics. The error, which may elsewhere also be referred to as a delta, may be determined for surgeries for which the actual surgery duration is known, for example for a set of surgeries which were previously performed within a hospital, e.g., within the last month(s) or year(s). By way of example, the following example refers to a set of cardiothoracic surgeries performed within a particular timeframe and within one hospital, on which the systems and methods described in this specification were evaluated. The deltas may for example be evaluated for all surgeries within the set, or for relevant sub-categories of surgeries. For example, the performance evaluation may distinguish between elective and acute surgeries and/or between medical specialties.

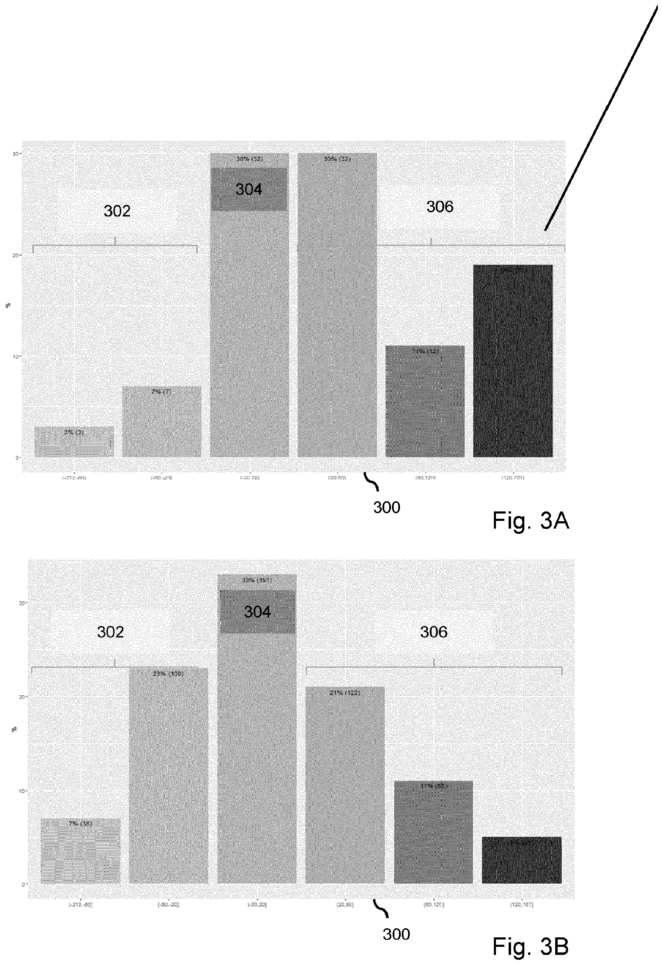

The deltas may in general be expressed in various ways, for example as a Root Mean Square Error (RMSE) or Mean Absolute Error (MAE). However, while such error metrics are well-known in the field of data analysis, they are not intuitive for use by clinical staff and planners. Further, these error measures do not distinguish between positive and negative time deltas. Instead, or additionally, a performance metric may be used which characterises whether the time difference between the predicted surgery duration and the actual surgery duration is positive or negative, as well as a degree of the difference. For example, such a performance metric may categorize the time difference into a number of categories, as for example explained with reference to Figs. 3A, 3B and elsewhere.

With continued reference to Fig. 2, in a second step 232, the surgeries may be categorized based on the deltas and then in a third step 234 visualized. Figs. 3A-3B show an example of such a visualization, which is further described below. In some embodiments, where the deltas for multiple predictive models are determined and visualized, a best predictive model may be selected in a fourth step 236, for example to be used in surgery planning. In other embodiments, based on the visualization, a user may decide that the deltas exceed an acceptability threshold and may use the SDP to generate a new predictive model, which functionality is further explained following Figs. 3A-3B.

Fig. 3A shows, for the aforementioned set of cardiothoracic surgeries, the performance of a conventional predictive model in predicting surgery duration for acute surgeries while Fig. 3B shows the performance for elective surgeries. The performance in both figures is expressed along the vertical axis as the "percentage (number)" of surgeries in each respective category on the horizontal axis 300. The categories may correspond to respectively a time range of more than 60 minutes ahead of schedule, a time range of 60 to 20 minutes ahead of schedule, a time range of 20 minutes ahead and 20 minutes behind, a time range of 20 to 60 minutes behind schedule, a time range of 60 to 120 minutes behind schedule and a time range of above 120 minutes behind schedule. Such categorization, however, is merely exemplary, and any other categorization may be used instead.

For example, as also shown in Figs. 3A and 3B by way of reference numerals 302-306, the individual time ranges may also be aggregated into three main categories, being 'ahead of schedule' 302, 'on time' 304 and 'behind schedule' 306, with for acute surgeries, 10% of surgeries being ahead of schedule, 30% on time and 60% behind schedule, and for elective surgeries, 30% being ahead of schedule, 33% on time and 37% behind schedule. Accordingly, 37% of all elective surgeries and 60% of all acute surgeries within the set of cardiothoracic surgeries are thus considered behind schedule compared to the prediction of the surgery duration by the conventional predictive model.

With continued reference to Fig. 2, the SDP 200 may be used to generate a predictive model for predicting the surgery duration, for example if clinical staff and/or planners are not satisfied with the performance of the current (conventional) predictive model. Generating a new predictive model may involve the following steps 210-218 as shown in Fig. 2. In a first step 210, medical data may be accessed and split into different parts. The accessed medical data may comprise records of surgeries, with each respective record being indicative of at least a type of surgery and a surgery duration. For example, medical data of the aforementioned cardiothoracic surgeries may be accessed, for example via a Hospital Information System (HIS). The medical data may then be partitioned into parts, e.g., a first part (e.g., 50% of records) for training predictive model(s), a second part (e.g., 25%) for testing the performance of the predictive model(s), including ensembles thereof, and a third part (e.g., 25%) for use in selecting a best performing model. It is noted that this step may be performed for all surgeries, or within a subset of all surgeries. For example, in some embodiments, separate predictive models may be generated for elective and acute surgeries, in which case the data splitting step may be applied only to the data representing the elective surgeries or only to the data representing acute surgeries.

In a second step 212, features may be extracted from the medical data. For example, some types of features may characterize the surgery while other types of features may characterize the patient. Examples of the former type of features are given in Table 1 below, while examples of the latter type of features are given in Table 2 below. It is noted that typically, all features are available before the surgery is performed, e.g., at the point of surgery planning, and typically even before the patient is hospitalized.

**Table 1: Features based on surgery characteristics**

| **Features** | | **Examples of values** |
|---|---|---|
| Surgeon ID | unclustered | ID_6791, ID_86 |
| | clustered | - Hierarchical clustering - ID_C1, ID_C2 |
| Surgeon type | categorical | Attendings vs. Residents |
| Surgery Procedures (Px) | unclustered | CABG, AVR, etc. |
| | clustered | According to: |
| | | - medical subspecialties |
| | | - Hierarchical clustering - Px_C1, Px_C2 |
| Ave Px time per surgeon | | 183 min, 316 min |
| Number of Px | categorical | Single, double, multiple (>=3) |
| | discrete | 1, 2, 3, 4, 5, ... |
| Surgery urgency | categorical | Acute, Elective |
| Post OR type of bed | categorical | ICU, PACU, general ward (GW) |

**Table 2: Features based on patient characteristics**

| **Features** | | **Examples of values** |
|---|---|---|
| Age | discrete | 67 years, 86 years |
| | categorical | [18-29], [30-44], [45-69], ... |
| Gender | categorical | Male vs. Female |
| BMI | categorical | Underweight, Normal, Overweight, Obese |
| ASA score | discrete | 1, 2, 3, 4, 5 |
| Medication numbers | discrete | 1,2, ...,22 |
| | categorical | [1, 5], [6-10], [11-15], [16++] |
| Creatinine levels | categorical | renal failure, severe decrease, moderate decrease, mild decrease, normal |

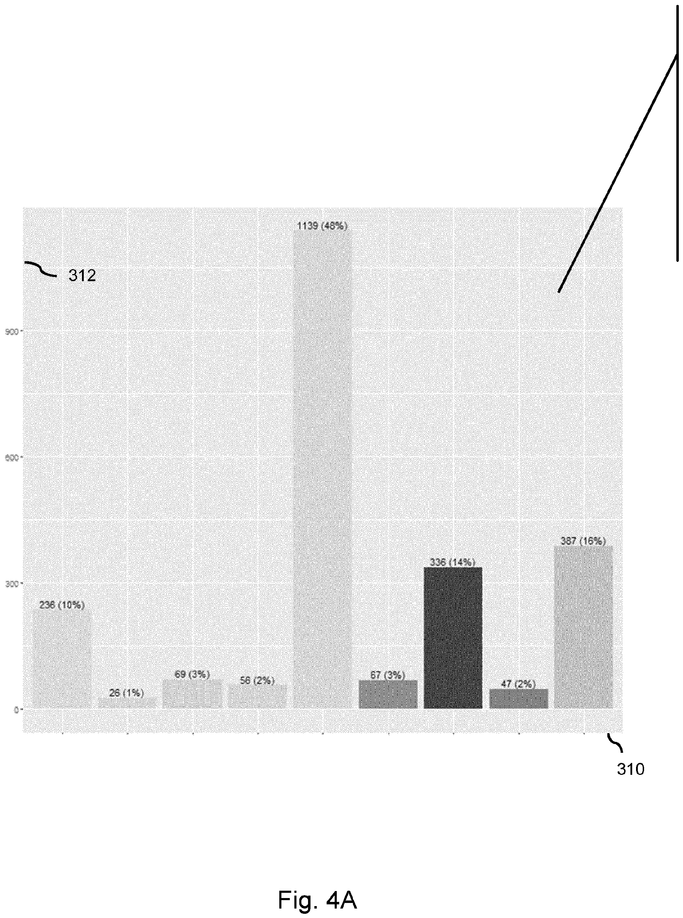

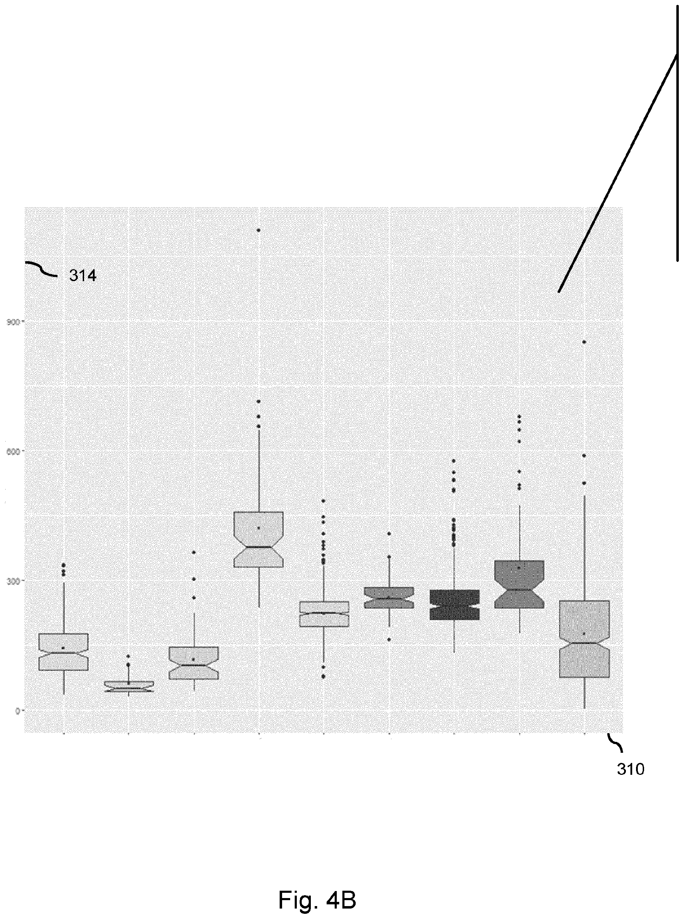

Figs. 4A, 4B illustrate that the type of surgical procedure may correlate with surgery duration. More specifically, Fig. 4A shows along the horizontal axis 310 a grouping of cardiothoracic surgeries per medical sub-speciality and along the vertical axis 312 the number of such surgeries within the training data. These medical sub-specialities are, from left to right, 1) lungs, 2) other, 3) pectus, 4) vascular, 5) CABG involving one artery, 6) CABG involving more than one artery, 7) AVR, 8) AVR-Aorta, and 9) heart - others. Fig. 4B shows the medical sub-specialities of Fig. 4A along the horizontal axis 310 in the same order as described for Fig. 4A and the surgery duration along the vertical axis 314, where it is evident that the actual surgery duration is dependent of the type of surgical procedure.

After having extracted a set of features from the training data, the feature selection may be performed, by which a set of features may be selected from the larger set of extracted features, which set of features is most predictive of the surgery duration. For the feature selection, any known feature selection technique may be used. In a specific example, a two-step feature selection technique may be used. For example, the SDP may first perform univariate inferential analysis to investigate how well each of the extracted features can predict the surgery duration. Features that are not statistically significant predictors (e.g., p-values > 0.05) may be dropped, see for example Table 3 below in which it is shown that gender is not a statistically significant predictor for surgery duration in this particular instance of medical data, i.e., this particular set of cardiothoracic surgeries.

**Table 3: Feature selection - univariate analysis**

| **Univariate inferential analysis by linear regression models for surgery duration** | | | |
|---|---|---|---|
| **Pt char, n (%)** | **p (< 0.05)** | **Pt char, n (%)** | **p (< 0.05)** |
| **Age category** | p < 0.001 | **ASA score** | p = 0.0385 |
| 18-29 years, 67 (3%) | | 1 cat, 33 (1%) | |
| 30-44 years, 63 (3%) | | 2 cat, 127 (5%) | |
| 45-59 years, 477 (20%) | | 3 cat, 1032 (44%) | |
| 60-74 years, 1265 (54%) | | 4 cat, 665 (28%) | |
| 75+ years, 491 (21%) | | 5 cat, 9 (0%) | |
| | | Unknown, 497 (21%) | |

| **Gender** | p = 0.899 | **Meds number** | p = 0.0041 |
|---|---|---|---|
| M, 1777 (75%) | | Range [0, 22] | |
| F , 586 (25%) | | | |

| **BMI category** | p < 0.001 | **Creatinine levels** | p = 0.01592 |
|---|---|---|---|
| Unknown, 365 (15%) | | renalFailure, 9 (0%) | |
| Underweight, 20 (1%) | | severeDecr, 120 (6%) | |
| Normal, 543 (23%) | | modDecr, 166 (7%) | |
| Overweight, 923 (39%) | | mildDecr, 1013 (43%) | |
| Obese, 512 (22%) | | normal, 759 (32%) | |
| | | unknown, 296 (12%) | |

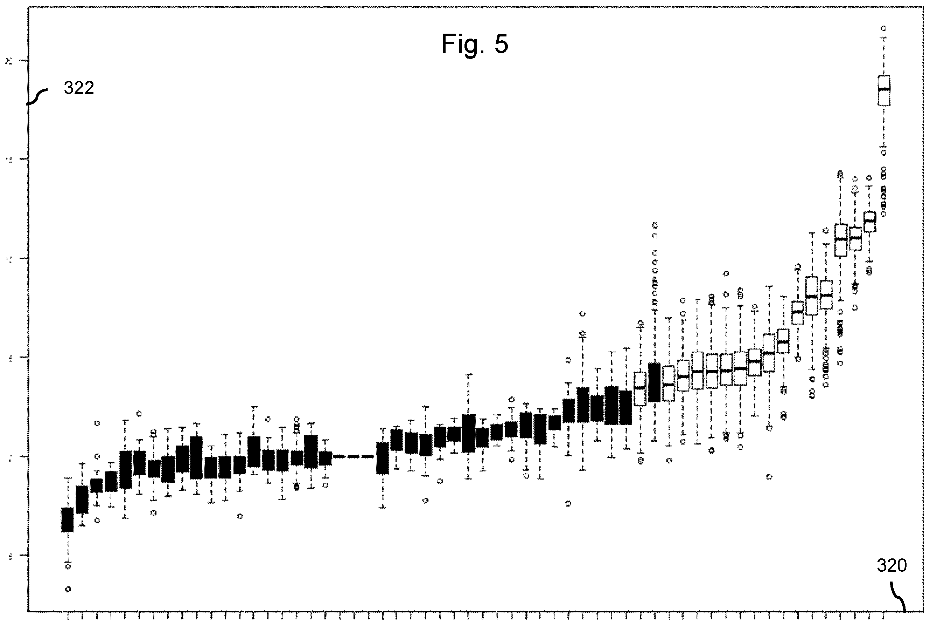

Secondly, the SDP may perform multivariate inferential analysis, for example using the Boruta algorithm based on Random Forest ML technique, which may categorize features into important, tentative, and unimportant features. An example of the output of the Boruta algorithm is visualized in Fig. 5, where the features (not separately identified) extracted from the training data are shown along the horizontal axis 320 and the feature importance 322 is shown along the vertical axis, with important and tentative features being shown in white and unimportant features being shown in black. A set of features may then be selected from the important features and optionally from the tentative features.

With continued reference to Fig. 2, in a third step 214, a number of predictive models may be trained using the set of features in the training data as input and the surgery duration as prediction target. The predictive models may for example include models based on linear regression (LM), which is a linear model, and random forest (RF) and/or gradient boosting (GB), both non-linear models. The SDP trains these predictive models on the training data. In some embodiments, the surgery urgency (acute/elective) may be a feature in the training, while in other embodiments, the training may be separate for acute and elective surgeries, resulting in dedicated predictive model(s) for acute and elective surgeries.

Having trained the predictive models, in a fourth step 216, a performance of each of the predictive models in predicting the surgery duration may be evaluated. For that purpose, a second part of the medical data may be used, which part may elsewhere also be referred to as test data. Such performance evaluation may for example make use of the traditional RMSE and MAE errors, which yield the performance numbers listed in Table 4 below, in which separate models were trained for elective and acute surgeries. Preferably, however, the performance evaluation comprises using a performance metric which characterises the time difference between the predicted surgery duration and the actual surgery duration. For example, the performance metric may indicate whether the difference between the predicted surgery duration and actual surgery duration is positive or negative and the magnitude of the difference. In some embodiments, the difference may also be categorized in several categories, e.g., as shown in Figs. 3A, 3B on the horizontal axis 300.

**Table 4: Predictive model performance. ^{∗} LM, RF, GB predictive models trained on cardiothoracic elective surgeries only. ^{∗∗} LM, RF, GB predictive models trained on cardiothoracic acute surgeries only.**

| Models | | Elective^{∗} | | Acute^{∗∗} | |
|---|---|---|---|---|---|
| | | **RMSE** | **MAE** | **RMSE** | **MAE** |
| Current | | 0.9894 | 0.7080 | 1.8740 | 1.2210 |
| New models | LM | 0.8375 | 0.6464 | 0.9246 | 0.6561 |
| | RF | 0.8278 | 0.6213 | 0.9189 | 0.6054 |
| | GB | 0.7994 | 0.6075 | 0.8910 | 0.6165 |

With continued reference to Fig. 2, in a fifth step 218, one or more ensembles may be created from the predictive models. These ensembles may be created by ensemble learning, for example using techniques such as blending (e.g., a form of stacking) and/or by aggregating the predictions of individual predictive models in a way that obtains an improved prediction of the surgery duration. Note that all predictive models may be used as input to the ensemble learning, or only selected models, e.g., those which perform best according to the performance metric. In some examples, if the individual predictive models have similar performance, ensembles of all possible combinations may be created. Each ensemble may be embodied by a separate model. It is noted that, typically, the ensemble learning may be performed using the test data, in that the ensemble model may be generated to optimize the performance metric on the test data.

Once the ensemble model(s) are generated, the SDA may again perform its steps 230-236 to quantify the performance improvement over the current predictive model. If there are multiple predictive models, for example a set of ensemble models or a combination of individual predictive models and ensemble models, the performance improvement may be evaluated for each of these models. For the performance evaluation, the aforementioned performance metric may be used which uses categorization with clinical meaning such as 'behind-schedule', 'on time', 'ahead of schedule'. Additionally, or alternatively, known error metrics such as the RMSE/MAE may be used in the evaluation. In general, the performance evaluation may be on a third part of the medical data, which third part is elsewhere also referred to as validation data. The best performance model may then be selected for subsequent inferential use, for example for use as a predictor in actual surgery planning.

For the medical data of the set of cardiothoracic surgeries, the best predictive model for surgery duration of acute surgeries was an ensemble model, namely LM + GB blended by RF, highlighted in Table 5 below. This ensemble model reduced the number of surgeries 'behind-schedule' by 28% (from 60% to 32%) and increased the surgery 'on-time' by 15% (from 30% to 45%). The number of surgeries 'ahead-of-schedule' was increased by 13% (from 10% to 23%), which may be considered to be an acceptable trade-off.

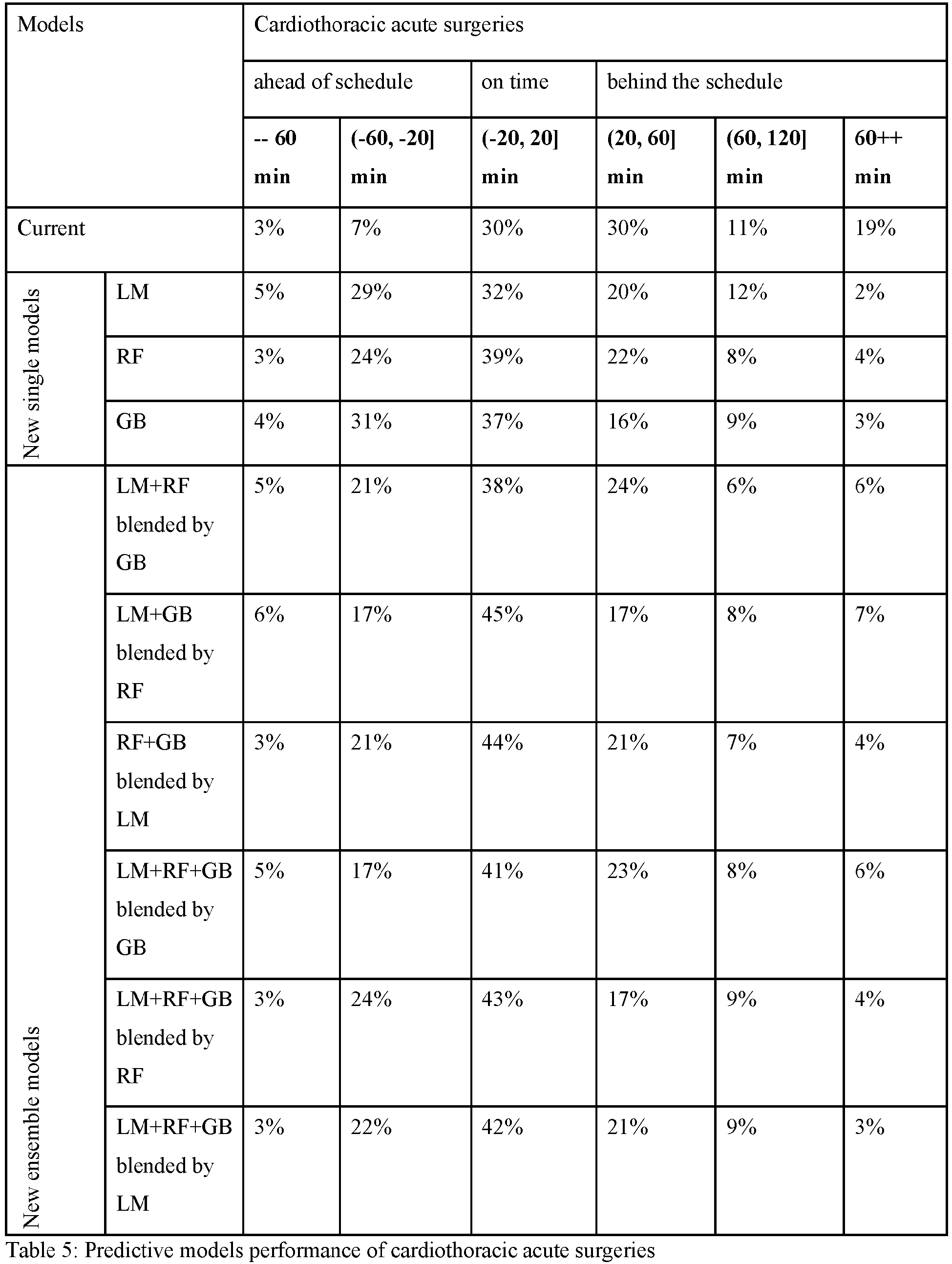

The best predictive model for surgery duration of elective cardiothoracic surgeries was also an ensemble model, namely the combination of LM + RF + GB blended by LM, highlighted in Table 6. This ensemble model reduced the number of surgeries 'behind-schedule' by 9% (from 37% to 28%) and increased the surgery 'on-time' by 5% (from 33% to 38%). The number of surgeries 'ahead-of-schedule' was increased by 4% (from 30% to 34%), which again may be considered to be an acceptable trade-off.

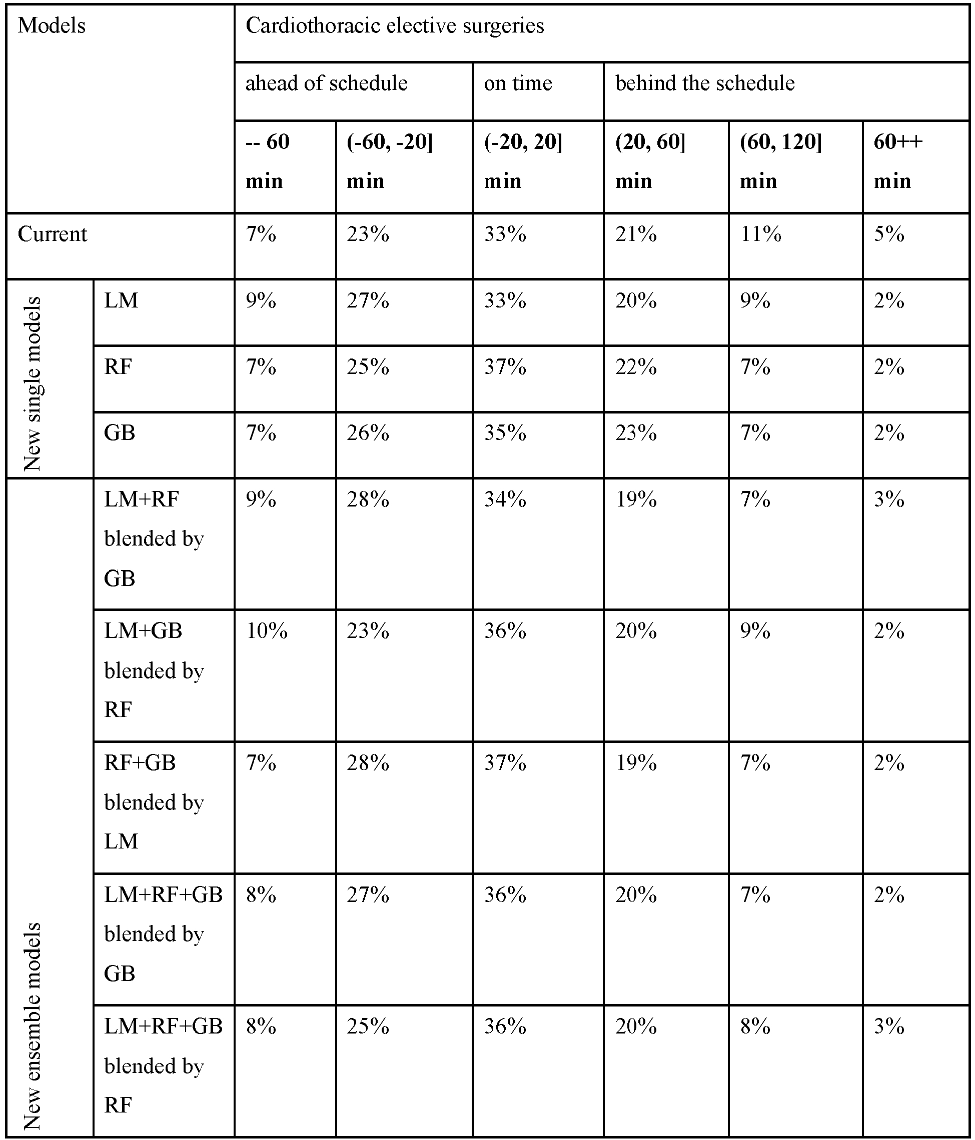

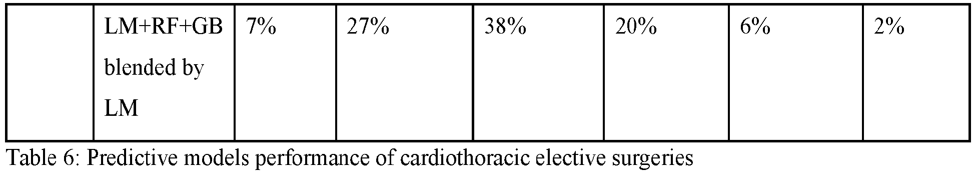

**Table 7: Surgery duration improvements for elective and acute surgeries. ^{∗} Predictive model (LM+RF+GB by LM) trained on cardiothoracic elective surgeries only. ^{∗∗} Predictive model (LM+GB by RF) trained on cardiothoracic acute surgeries only.**

| Surgeries | Cardiothoracic elective^{∗} | Cardiothoracic acute^{∗∗} |
|---|---|---|
| "behind the schedule" | reduced 9 % (from 37% to 28%) | reduced 28% (from 60% to 32%) |
| "on time" | increased 5% (from 33% to 38%) | increased 15% (from 30% to 45%) |
| "ahead of schedule" | increased 4% (from 30% to 34%) | increased 13% (from 10% to 23%) |

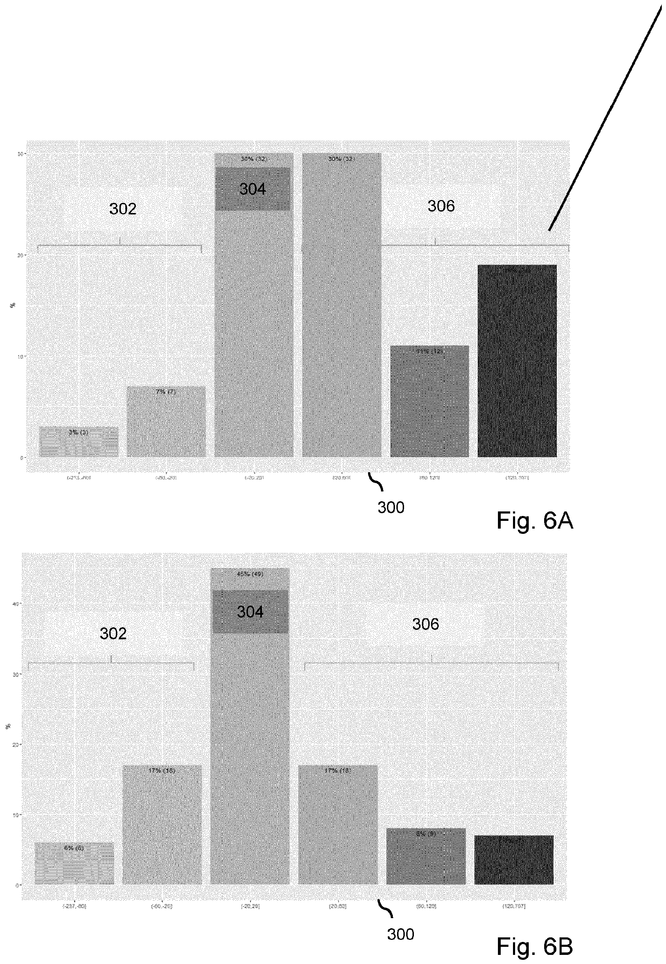

Fig. 6B also provides a graphical representation of the performance of the best ensemble model for acute surgeries, namely the aforementioned LM + GB blended by RF. It can be seen that 23% of surgeries are ahead of schedule, 45% on time and 32% behind schedule. For comparison purposes, Fig. 6A is the same as Fig. 3A, showing the performance of a conventional predictive model in predicting surgery duration for acute surgeries, i.e., 10% of surgeries ahead of schedule, 30% on time and 60% behind schedule.

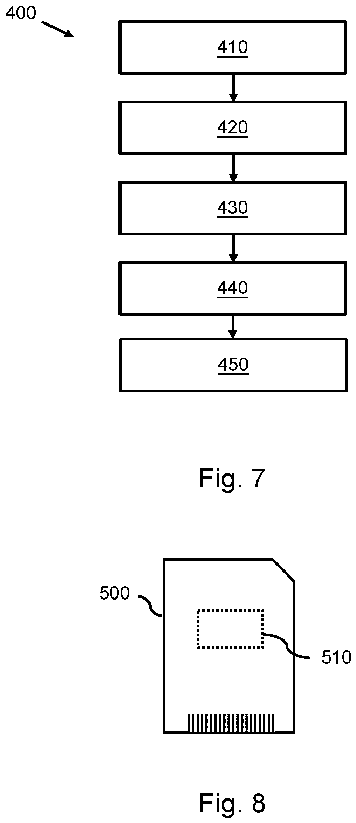

Fig. 7 shows a block-diagram of computer-implemented method 400 for generating a predictive model for predicting a surgery duration. The method 400 may correspond to an operation of the system 100 of Fig. 1. However, this is not a limitation, in that the method 400 may also be performed using another system, apparatus or device.

The method 400 is shown to comprise, in an operation titled "ACCESSING MEDICAL DATA", accessing 410 medical data as described elsewhere in this specification, and generating a predictive model for predicting the surgery duration by, in an operation titled "IDENTIFYING FEATURES IN MEDICAL DATA", using a feature selection technique to identify 420 a set of features in training data, which set of features is predictive of the surgery duration, wherein the training data comprises a first part of the medical data, in an operation titled "TRAINING PREDICTIVE MODELS", training 430 a number of predictive models using the set of features in the training data as input and the surgery duration as prediction target, wherein the predictive models include at least a linear predictive model and a non-linear predictive model, in an operation titled "EVALUATING PERFORMANCE OF PREDICTIVE MODELS", using a second part of the medical data, evaluating 440 a performance of each the predictive models in predicting the surgery duration, wherein the evaluating of the performance comprises using a performance metric which characterises a time difference between a predicted surgery duration and an actual surgery duration, and in an operation titled "GENERATING AND OUTPUTTING ENSEMBLE MODEL", based on the performance of the predictive models, generating 450 an ensemble model which combines at least two of the predictive models and outputting the ensemble model for use in predicting the surgery duration. It will be appreciated that in general, operations of method 400 of Fig. 7 may be performed in any suitable order, e.g., consecutively, simultaneously, or a combination thereof, subject to, where applicable, a particular order being necessitated, e.g., by input/output relations. Operations may also be performed as part of other operations.

The method may be implemented on a computer as a computer implemented method, as dedicated hardware, or as a combination of both. As also illustrated in Fig. 8, instructions for the computer, e.g., executable code, may be stored on a computer readable medium 500, e.g., in the form of a series 510 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 8 shows a memory device 500.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system for generating a predictive model for predicting a surgery duration, comprising:
- an input interface for accessing medical data, the medical data comprising records of surgeries, wherein a record is indicative of at least a type of surgery and a surgery duration;
- a processor subsystem configured to generate a predictive model for predicting the surgery duration by:
- using a feature selection technique to identify a set of features in training data, which set of features is predictive of the surgery duration, wherein the training data comprises a first part of the medical data;
- training a number of predictive models using the set of features in the training data as input and the surgery duration as prediction target, wherein the predictive models include at least a linear predictive model and a non-linear predictive model;
- using a second part of the medical data, evaluating a performance of each the predictive models in predicting the surgery duration, wherein the evaluating of the performance comprises using a performance metric which characterises a time difference between a predicted surgery duration and an actual surgery duration;
- based on the performance of the predictive models, generating an ensemble model which combines at least two of the predictive models; and
- outputting the ensemble model for use in predicting the surgery duration.

2. The system according to claim 1, wherein the performance metric characterises whether the time difference between the predicted surgery duration and the actual surgery duration is positive or negative and a degree of the difference.

3. The system according to claim 2, wherein the performance metric categorizes the time difference into a number of categories, wherein the categories include at least a first category indicating that the predicted surgery duration exceeds the actual surgery duration by a first margin and a second category which indicates that the predicted surgery duration is less than the actual surgery duration by a second margin.

4. The system according to claim 2 or 3, wherein in evaluating the performance, a positive time difference between the predicted surgery duration and the actual surgery duration is weighted differently than a negative time difference.

5. The system according to any one of claims 1 to 4, further comprising a user interface subsystem comprising a user input interface to a user input device for receiving user input and a display output to a display for displaying output of the system, wherein the processor subsystem is configured to, using the user interface subsystem:
- enable a user to evaluate the performance of a previous predictive model for predicting the surgery duration using the performance metric;
- request a new predictive model to be generated by the system if the performance of the previous predictive model is deemed insufficient by the user or the system.

6. The system according to claim 5, wherein the processor subsystem is configured to, using the user interface subsystem, enable the user to further evaluate the performance of the new predictive model using the performance metric.

7. The system according to any one of claims 1 to 6, wherein the processor subsystem is configured to:
- generate two or more ensemble models which each combines at least two of the predictive models;
- using a third part of the medical data, evaluating a performance of the two or more ensemble models in predicting the surgery duration, wherein the evaluating of the performance comprises using the performance metric; and
- based on the performance of the two or more ensemble models, selecting the ensemble model for output.

8. The system according to any one of claims 1 to 7, wherein a record in the medical data is further indicative of one or more of: an identity of a surgeon, a clinical role of a surgeon, a type of surgical procedure, a surgical urgency, a type of post-surgery bed, an average surgery duration for a type of surgical procedure for a surgeon, and a number of surgical procedures performed during the surgery.

9. The system according to any one of claims 1 to 8, wherein the medical data further comprises patient data of patients of the respective surgeries.

10. The system according to claim 9, wherein the patient data is indicative of one or more of: an age, a gender, a body-mass index, an American Society of Anaesthesiology (ASA)-score, a number of medications taken, a number of comorbidities, and a creatine level, of a respective patient.

11. The system according to any one of claims 1 to 10, wherein the processor subsystem is configured to identify the set of features in the training data using multivariate inferential analysis, wherein the processor subsystem is further configured to use univariate inferential analysis as a filter to determine an input set of features to the multivariate inferential analysis, wherein the features of the input set of features are individually predictive of the surgery duration.

12. The system according to any one of claims 1 to 11, wherein the predictive models comprise one or more of: a linear regression-based model, a random forest-based model, and a gradient boosting-based model.

13. The system according to any one of claims 1 to 12, wherein a record in the medical data is indicative of whether a surgery is an elective surgery or an acute surgery, wherein the processor subsystem is configured to generate different predictive models for predicting the surgical duration of elective surgeries and for predicting the surgical duration of acute surgeries.

14. A computer-implemented method for generating a predictive model for predicting a surgery duration, comprising:
- accessing medical data, the medical data comprising records of surgeries, wherein a record is indicative of at least a type of surgery and a surgery duration;
- generating a predictive model for predicting the surgery duration by:
- using a feature selection technique to identify a set of features in training data, which set of features is predictive of the surgery duration, wherein the training data comprises a first part of the medical data;
- training a number of predictive models using the set of features in the training data as input and the surgery duration as prediction target, wherein the predictive models include at least a linear predictive model and a non-linear predictive model;
- using a second part of the medical data, evaluating a performance of each the predictive models in predicting the surgery duration, wherein the evaluating of the performance comprises using a performance metric which characterises a time difference between a predicted surgery duration and an actual surgery duration;
- based on the performance of the predictive models, generating an ensemble model which combines at least two of the predictive models; and
- outputting the ensemble model for use in predicting the surgery duration.

15. A transitory or non-transitory computer-readable medium comprising data representing a computer program, the computer program comprising instructions for causing a processor system to perform the method according to claim 14.
